# EUROPEAN PATENT APPLICATION

(11) **EP 0 966 932 A1**
(43) Date of publication of application: **29.12.1999**
(21) Application number: 98610019.6
(22) Date of filing: 23.06.1998
(51) Int. Cl.: A61F 5/02

(54) **Harness for fitting on the upper part of the body**

(71) Applicant: Goller, Gitte Lettee, 8600 Silkeborg (DK)
(72) Inventor: Petkovski, Slavi, 8600 Silkeborg (DK)
(74) Representative: Larsen, Hans Ole

(57) **Abstract**

With physical disabilities and with straining work, the body can require a support harness to relieve such loads which could result in inconveniences or deformities.

The harness according to the invention distinguishes itself by having an expedient positioning of straps (2, 3) around the shoulders (18), which ensures that the shoulders are not forced forwards, with a bad back and neck posture as a consquence.

The harness comprises a back-piece (1) from which two pairs of shoulder straps (2, 3) extend around the shoulders (18), and also a lower pair of straps (4) which extend around the waist (11). The harness is configured in such a manner that it does not impair the full freedom of movement of the arms (19).

## Description

### Prior art technique

The invention concerns a harness for fitting on the upper part of the body, around which it is secured by means of straps which extend around the body and are fastened at the front.

Harnesses of this kind are normally used as a support for persons with a physical disability which requires an external influence in the form of a relieving support.

The disability can be a disorder in the spinal column, collarbone or shoulder blade, which requires this external support in order to compensate for the lack of muscular strength, or as an aid in the counteracting of possible over-exertion of the joints or muscles.

An example of such a harness is known from the description of US patent no. 1,050,257, where the harness comprises two upper straps for placing around the shoulders, and with a lower strap for placing around the waist. The harness is provided at the back with a narrow back-piece which extends over the spinal column.

However, this known harness is not able to provide the support necessary for the shoulders, in that the back-piece is too narrow to provide the necessary counterpressure for the remaining straps, which means that the shoulders can not be held sufficiently firm in their straightened-up position. To this can be added that the harness is made of an elastic material, which does not provide a firm support, the reason being that the harness will yield to the movements of the body. Finally, since the harness must be fastened from the back, the fitting of the harness also requires a helper.

### Advantages of the invention

It is the object of the invention to redress These drawbacks and disadvantages, and at the same time to ensure an optimum aid, and according to the invention this is achieved when the harness comprises a back-piece of a length which corresponds to the distance from the neck and down to around the waist, and the breadth of which at the top corresponds to the distance between the shoulder blades, and to which back-piece there are secured two pairs of straps, the upper pair of which extend from the upper corners of the back-piece and over each shoulder, and where the lower straps extend from the middle of the back-piece's side edges and under each arm, and also a further pair of straps which extend from the lowermost side edge of the back-piece and around the waist, and where the straps can be held together in pairs at the front of the upper part of the body.

In a surprisingly simple and natural manner, there is hereby achieved a support with straps which are placed in such a way that the harness fitted on the upper body does not have a restricting effect on the normal use of the muscles, the reason being that the straps extend substantially across the large movement muscles.

A posture with bowed back is destructive for the muscle disks, and can give rise to a pinching of the nerves. This results in an accumulation of fluid around the spinal column, and with the risk of considerable pain.

By configuring the back-piece of the harness with such a breadth that it supports the upper chest vertabrae between the shoulder blades, the harness prevents the upper body from sinking down and falling forwards.

Since the harness can also be tightened relatively hard to the body, the support becomes very effective without any sacrifice regarding mobility in neck and arms.

As disclosed in claim 2, by letting the upper pair of straps extend around the shoulders, i.e. around the collarbone and the shoulder blade, the shoulder joint is left free, which gives unimpaired mobility in the shoulder joint. Moreover, tightened straps will draw the shoulders back to achieve a good, upright spinal column and neck. This is of decisive importance for persons who must work more or less permanently with the hands in front of the body. These persons can be keyboard operators, drivers and other people who, during their work, easily slump down into a bowed posture. Furthermore, the loading of the neck and the shoulder region, which arises in connection with the lifting of burdens, is taken up by the harness and transferred to the back, where the load is distributed.

As disclosed in claim 3, by producing the harness in a material such as terry towelling, the harness is made comfortable and also washable, and the material can possibly be able to be strengthened with inserts or with plastic fibre for the reduction of its dimensions.

As disclosed in claim 4, the insertion of a heat-insulating layer in the back-piece will result in an expediently higher body temperature, which increases the circulation of blood in the back of the user.

As disclosed in claim 5, by providing the straps with a velcro-band, the straps are easy to open and close and, since they can be placed in any position, they can easily be adjusted.

Finally, as disclosed in claim 6, it is expedient to use the harness to ensure a good posture.

### The drawing

An example embodiment of the harness will now be described in more detail with reference to the drawing, where
- fig. 1: shows a harness according to the invention fitted on a person seen from the front, and
- fig. 2: shows the harness and the person seen from the back.

### Description of the example embodiment

The drawing shows an example of a harness fitted on the upper body of a person who is depicted with the outer muscles and skeletal parts exposed.

The harness is made of material and is configured as a back-piece 1, see fig. 2, which is shaped in such a way that at the top it extends across the spinal column out to the shoulder blades and under the neck 10. From here, the back-piece 1 extends downwards to a position over the waist 11.

The back-piece 1 is preferably configured as a laminate in which there is inserted a heat-insulating layer surrounded by e.g. terry towelling. This ensures a reduced dissipation of heat from the back and herewith a higher surface temperature over the back region.

As shown in fig. 2, three pairs of straps extend from each side of the back-piece 1, i.e. an upper strap 2 which extends from the upper corners, a centre strap 3 which extends from the middle of the back-piece 1, and a lower strap 4 which extends from the lower side edges of the back-piece.

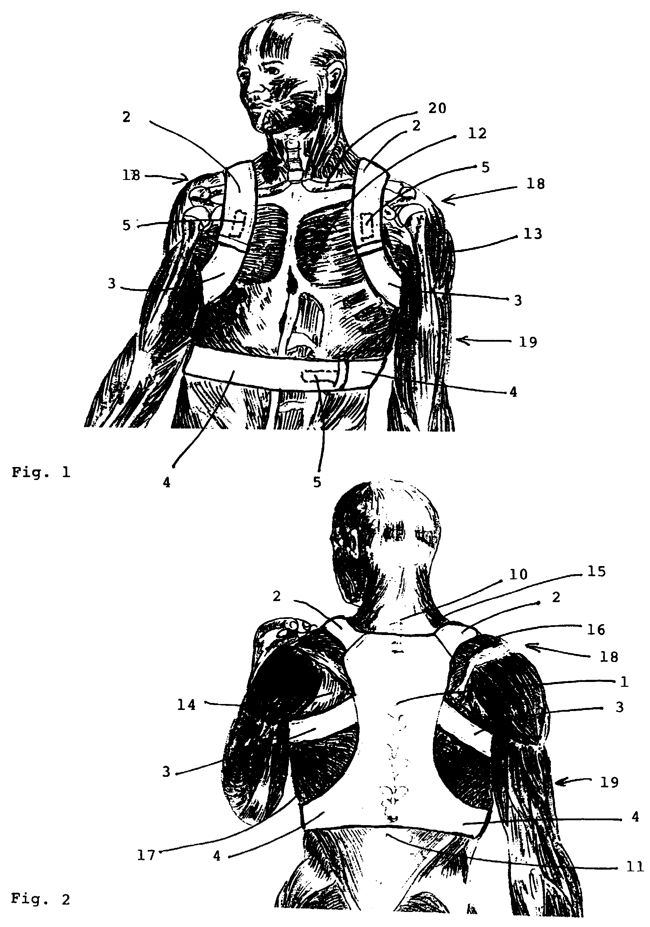

As shown in fig. 1, these straps are of such a length that they can overlap one another on the front of the upper body.

On the ends of the straps there are sewn velcro-type bands with hooks on the overlying straps and hoops on the underlying straps.

There is hereby achieved a reliable closing in that the back-piece 1 is placed on the person's back, after which the upper straps 2 are led over the shoulders and the collarbone, while the lower straps 3 are led under the arms 19 in the armpits and forward to where the two straps are then fastened together by means of the velco-bands 5, as shown in fig. 2.

The lowermost straps 4 are led around the waist 11 and fastened at the front by means of the velcro-band, as shown in fig. 1.

The straps are then suitably tightened and fastened together, which results in a relieving of the muscles, which in practice means that these can remain passive and thus do not load the body.

Furthermore, it is ensured that the mobility of the arms is retained, and that the shoulder movements are limited only by the freedom of movement for which the straps are adjusted. Since in this manner the muscles are relieved, the consumption of oxygen is reduced and herewith also the liberation of carbon dioxide, which in practice means that the large shoulder muscle 16, see fig. 2, is relieved, which results in a considerable reduction in the muscles' work and which is experienced as a rest. Consequently, a considerable degree of relief is achieved by means of this harness.

The whole of the harness is configured in such a manner that it is of the least possible inconvenience for the movements. It should be noted that all of the straps shown in the drawing extend in a substantially transverse manner in relation to the large muscles, which means that the function of the muscles is not impaired by the harness.

Moreover, the straps extend around the shoulder blade in such a way that the shoulder blade is held back in an upright position, even when the upper body is bowed forwards. This is achieved by the upper body being controlled in the remaining straps 2, 3, and these straps prevent a bending-over of the shoulder blades, in that the shoulders are held back and retain their upright position regardless of the position of the upper body.

In addition to ensuring a good upright posture, the harness also contributes towards the transfer via the straps of the loads on the arms/shoulders to the back-piece, whereby the load is distributed across the back where it can be absorbed more easily.

In this manner there is achieved a hitherto-unknown degree of support for the upper body, and moreover without this having influence e.g. on the free movement of the arms in relation to the upper body.

## Claims

1. Harness for fitting on and fastening around the upper body by means of straps which extend around the body and are fastened together at the front, **characterized** in that the harness comprises a back-piece (1) with a length which corresponds to the distance from the neck (10) and down to around the waist (11), and the breadth of which at the top corresponds to the distance between the shoulder blades, and to which back-piece (1) there are secured two pairs of straps, the upper pair (2) of which extend from the upper corners of the back-piece (1) and over each shoulder (18), where the lower straps (3) extend from around the middle of the side edges of the back-piece (1) and under each arm (19), and also a pair of straps (4) which extend from the lowermost side edge of the back-piece (1) and around the waist (11), and where the straps (2,3 and 4) can be fastened together in pairs at the front of the upper body.

2. Harness according to claim 1, **characterized** in that the upper pair of straps (2,3) extend around the shoulders (18).

3. Harness according to claims 1 and 2, **characterized** in that the back-piece (1) and the straps (2,3 and 4) are produced from the same material.

4. Harness according to claims 1 and 2, **characterized** in that the back-piece (1) is configured as a pillow with a heat-insulating insert.

5. Harness according to any of the foregoing claims, **characterized** in that the pairs of straps (2,3 and 4) are provided with velcro-type fasteners (5) with hooks and loops respectively.

6. Use of a harness according to claims 1-5, **characterized** in that the harness according to (1-5) is used to ensure an appropriate anatomical bodily posture with a straight neck and back and with the shoulders of the wearer drawn backwards.
